(19) Europäisches Patentamt
European Patent Office
Office européen des brevets

(11) Numéro de publication : **0 041 908**
**B1**

(12) **FASCICULE DE BREVET EUROPÉEN**

(45) Date de publication du fascicule du brevet : **29.02.84**

(51) Int. Cl.³ : **C 07 C 87/60**, C 07 C 85/24

(21) Numéro de dépôt : **81420084.6**

(22) Date de dépôt : **03.06.81**

(54) **Procédé de fabrication de la dichloro-2,6-nitro-4-aniline et composé obtenu par ce procédé.**

(30) Priorité : **06.06.80 FR 8013021**

(43) Date de publication de la demande : **16.12.81 Bulletin 81/50**

(45) Mention de la délivrance du brevet : **29.02.84 Bulletin 84/09**

(84) Etats contractants désignés : **AT BE CH DE FR GB IT LI LU NL SE**

(56) Documents cités :
**DE-A- 432 801**
**FR-A- 2 368 465**
**CHEMICAL ABSTRACTS, vol. 90, no. 15 09-04-1979, réf. 121176d, page 605, Columbus, Ohio, US**
**CHEMICAL ABSTRACTS, vol. 51, no. 7 10-04-1957, réf. 5008e Columbus, Ohio, US, L.M. LITVINENKO et al.: "Synthesis of some halogen-containing amino and nitro derivatives of biphenyl"**

(73) Titulaire : **RHONE-POULENC AGROCHIMIE**
**14-20, rue Pierre Baizet**
**F-69009 Lyon (FR)**

(72) Inventeur : **Aubouy, Michel**
**59, Boulevard Beauséjour**
**F-75016 Paris (FR)**
Inventeur : **Hamel, Pierre**
**3, Rue Voltaire**
**Saint Aubin-Les-Elbeuf F-76410 Cleon (FR)**
Inventeur : **Molin, Marc**
**6, Rue de Suffren**
**F-93330 Neuilly s/Marne (FR)**

(74) Mandataire : **Chretien, François et al**
**RHONE-POULENC AGROCHIMIE BP 9163**
**F-69263 Lyon Cedex 1 (FR)**

**EP 0 041 908 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Procédé de fabrication de la dichloro-2,6-nitro-4-aniline et composé obtenu par ce procédé

La présente invention a pour objet un nouveau procédé de fabrication de la dichloro-2,6-nitro-4-aniline.

Ce composé est connu notamment comme intermédiaire de la dichloro-3,5-aniline, elle-même utilisée pour la fabrication de divers produits phytosanitaires en particulier fongicides.

La préparation de la dichloro-2,6-nitro-4-aniline par chloration de la paranitroaniline à l'aide de chlore gazeux, en milieu chlorhydrique aqueux, a été d'abord étudiée par Flurscheim (cf. J. Chem. Soc. 93, 1772-5 (1908)). Cette étude montre que, lorsqu'on opère à froid et en milieu moyennement concentré (l, 8N), le rendement est faible (37 %), mais qu'il peut être sensiblement augmenté en travaillant à l'ébullition et en milieu chlorhydrique dilué (0,25 N).

Cette technique de laboratoire a été reprise dans de nombreuses références ultérieures relatives à des procédés plus industriels. Ceux-ci font appel, en plus ou à la place de l'acide chlorhydrique, à un autre acide fort tel que l'acide sulfurique ou l'acide acétique ou encore à un tiers solvant. Cependant ces procédés n'ont pas un rendement suffisant, donnent un produit de qualité médiocre, ou posent des problèmes de séparation et de récupération à fins de recyclage des eaux-mères.

En outre, le document CS-A-175 295 (VANC, VACLAV et al.) (15-11-1978) décrit la chloration de la paranitraniline par action d'eau oxygénée en milieu chlorhydrique 5N, à une température de 38-45 °C.

Le document FR-A-2 368 465 de BASF décrit la chloration, notamment de la paranitraniline, à l'aide d'un hypochlorite alcalin, à une température ne dépassant pas 60 °C et en milieu acide plus dilué que celui de la présente invention.

Cependant, comme la demande en dichloro-2,6-nitro-4-aniline va en croissant, il devient de plus en plus nécessaire de trouver un procédé de fabrication industriel, c'est-à-dire produisant un produit de bonne qualité, en particulier de bonne filtrabilité et dans des conditions moins polluantes.

La présente invention a pour but de fournir un procédé amélioré et simplifié par rapport aux techniques antérieures. Elle concerne également la forme particulière du produit telle qu'on l'obtient par ce procédé.

Plus précisément, l'invention a pour objet un procédé de fabrication de la dichloro-2,6-nitro-4-aniline par chloration, avec du chlore gazeux, de la paranitroaniline en solution aqueuse d'acide chlorhydrique, à l'ébullition, caractérisé en ce que, la réaction est effectuée à une température de 95 à 110 °C, la concentration du milieu en acide chlorhydrique étant comprise entre 4 et 7,5 N et, de préférence, entre 4,5 et 6 N.

Cette réaction s'effectue selon le schéma :

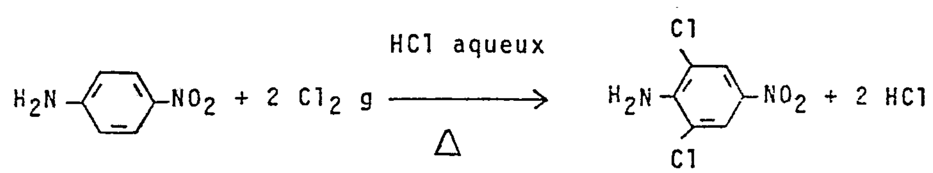


Dans les conditions de la réaction, la paranitroaniline est très soluble dans le milieu. On obtient à froid une suspension qui est dissoute par chauffage à la température retenue. On introduit alors un courant de chlore dans le réacteur au moyen d'un tube plongeur, la température étant maintenue sensiblement constante. La quantité de chlore engagée est en général au moins stœchiométrique. Une quantité inférieure est néfaste au rendement. Un excès n'est pas néfaste mais n'améliore pas le rendement. En pratique, on travaille dans des quantités stœchiométriques mais un excès par rapport à la stœchiométrie, par exemple jusqu'à 50 % en mole convient également bien.

La concentration de départ du milieu en acide chlorhydrique doit être inférieure à la saturation, c'est-à-dire choisie entre 4 et 7,5 N et de préférence entre 4,5 et 6 N. La demanderesse a en effet constaté que, contrairement à l'enseignement de l'art antérieur, un milieu chlorhydrique concentré (en combinaison avec la température élevée) est favorable à l'obtention d'un rendement élevé. On travaille de préférence à la concentration d'une mole d'amine par litre d'acide chlorhydrique. Comme la réaction produit elle-même deux moles d'acide chlorhydrique, la normalité du milieu augmente d'environ deux unités au cours de la réaction, lorsqu'il n'y a pas de dégazage. Or il faut qu'en fin de réaction, le milieu ne devienne pas sursaturé, sinon l'acide excédentaire dégaze et concurrence le chlore dans son transfert vers le liquide, ce qui augmente la durée de réaction. C'est pourquoi, la normalité de saturation étant d'environ 9,5 N, la concentration du milieu en acide chlorhydrique en début de réaction sera d'au plus 7,5 N.

Par ailleurs, une concentration inférieure à 4 N n'est pas avantageuse car le rendement tombe alors en-dessous d'environ 75 %, ce qui est insuffisant pour un procédé industriel.

Une autre caractéristique du procédé selon l'invention, est d'opérer à une température élevée, de 95 à 110 °C, en pratique à la température d'ébullition du milieu selon la concentration choisie en acide chlorhydrique. La demanderesse a en effet constaté que, dans ces conditions, on obtient non seulement des rendements élevés mais que, de plus, le produit obtenu est d'une excellente filtrabilité, ce qui

raccourcit considérablement la durée de séparation de la dichloro-2,6-nitro-4-aniline. En particulier, les meilleurs résultats sont obtenus pour des températures comprises entre 105 et 110 °C, correspondant au voisinage du point d'ébullition de l'azéotrope du mélange acide chlorhydrique-eau. La taille des cristaux diminue avec la température, la limite inférieure se situant vers 95 °C si on ne veut pas augmenter considérablement la durée de réaction.

Selon un autre mode de réalisation de l'invention, on peut opérer avec une surpression. Notamment une légère surpression accélère le transfert du chlore dans la solution. On a observé, par exemple, qu'une surpression de 0,26 bar permet de réduire de 4 à 5 fois la durée de chloration, à 110 °C dans l'acide chlorhydrique 6 N au départ. On peut également travailler en autoclave à pression supérieure avec ou sans dégazage.

La dichloro-2,6-nitro-4-aniline, insoluble dans les conditions de réaction, précipite rapidement et progressivement jusqu'à la fin. Le précipité est filtré. Les eaux-mères sont essentiellement constituées d'une solution aqueuse d'acide chlorhydrique plus concentrée que la solution de départ. Elles peuvent donc, par simple dilution avec de l'eau, être immédiatement utilisées pour une nouvelle opération. Le précipité est ensuite lavé et isolé de manière usuelle.

Le produit obtenu se présente sous la forme de gros cristaux, d'une taille moyenne comprise entre 100 et 300 microns dans les conditions où l'on a opéré. Au sens de l'invention, on entend par « taille moyenne », la moyenne des plus grandes dimensions des cristaux d'un échantillon. Cette présentation particulière est nouvelle, puisque les cristaux du même produit obtenu par les procédés connus de chloration en milieu aqueux ont une taille moyenne environ dix fois moindre et une humidité double. Cette présentation permet d'abaisser le taux d'humidité du produit filtré. Ceci explique que le produit selon l'invention possède une remarquable filtrabilité qui permet d'augmenter considérablement la productivité d'une unité de fabrication donnée.

Le précipité contient, outre ce produit, de petites quantités de l'isomère dichloro-2,4-nitro-6-aniline et du dérivé monochloré, la chloro-2-nitro-4-aniline, qui ne sont pas habituellement séparés car utilisables ou non gênants dans la transformation ultérieure en dichloro-3,5-aniline. Les rendements bruts peuvent être supérieurs à 90 %, ceux en dichloro-2,6-nitro-4-aniline supérieurs à 80 % et pouvant atteindre 90 %.

Outre les bons rendements et l'excellente filtrabilité du produit, le procédé selon l'invention présente d'autres avantages comme la facilité de conduite de la chloration, la température se maintenant d'elle-même par le reflux, l'arrêt pratiquement total de l'absorption de chlore en fin de réaction, ce qui évite les surchlorations, l'absence de consommation d'acide chlorhydrique en raison du recyclage des eaux-mères, sans nécessité d'ajouter de l'acide neuf, enfin la réduction des effluents qui sont limités aux eaux de lavage.

Les exemples suivants illustrent des modes de réalisation du procédé selon l'invention.

## Exemple 1

Dans un réacteur muni d'un réfrigérant à reflux et d'une colonne d'absorption de gaz arrosée à la soude, on charge un mélange de 1 litre d'acide chlorhydrique 4,5 N pour une mole de paranitroaniline (138 g). La suspension est agitée et portée vers 105 °C et on introduit un courant de chlore dans l'appareil au moyen d'un tube plongeur en maintenant la température vers 105 °C. Au bout d'environ 15 minutes, il apparaît un précipité qui s'épaissit peu à peu. Après environ 2 heures, on réduit progressivement le débit de chlore jusqu'à ce qu'il ne soit plus absorbé. On introduit ainsi environ 2,2 moles de chlore par mole d'amine sur une durée totale de 3 à 4 heures.

Le mélange est refroidi vers 70-80 °C pour être filtré et lavé à l'eau. Les eaux-mères de filtration sont conservées pour la préparation des charges de l'opération suivante. Les eaux de lavage sont évacuées.

On obtient 190,5 g de produit sec par mole de paranitraniline chargée. Rendement = 92 %.

L'analyse du produit obtenu indique :

82 % de dichloro-2,6-nitro-4-aniline
1,5 % de dichloro-2,4-nitro-6-aniline et
5 % de chloro-2-nitro-4-aniline.

Les cristaux ont une taille moyenne d'environ 150-200 microns, ce qui représente environ 10 fois la taille des cristaux obtenus par la chloration dans les conditions habituelles. De même, la résistance spécifique du gâteau, caractéristique de la filtrabilité se trouve divisée par un facteur 5 à 10 par rapport aux autres procédés de chloration en milieu aqueux.

Le taux d'humidité du produit filtré et lavé est de 20 à 25 %, contre 40-45 % dans les conditions habituelles de chloration.

## Exemple 2

Les eaux-mères provenant de la filtration de l'opération précédente, soit environ 0,75 l d'acide chlorhydrique environ 6 N pour une mole d'amine chargée, sont complétées par de l'eau de façon à obtenir 1 litre d'acide chlorhydrique 4,5 N.

La chloration a lieu dans ce mélange comme dans l'exemple 1. L'opération de recyclage est répétée au moins dix fois sans influence sur le rendement et la qualité du produit obtenu.

Exemple 3

On opère comme dans l'exemple 1, mais en chargeant de l'acide chlorhydrique 6 N et on effectue la chloration à la température de 95 °C. Le produit est isolé comme dans l'exemple 1. On obtient 196,7 g de produit sec. Rendement = 95 %. L'analyse du produit obtenu indique :

88 % de dichloro-2,6-nitro-4-aniline
2 % de dichloro-2,4-nitro-6-aniline et
4 % de chloro-2-nitro-4-aniline.

Exemple 4

On opère comme à l'exemple 1 si ce n'est qu'on charge de l'acide chlorhydrique 6 N et qu'on chauffe à 110 °C, point d'ébullition de l'azéotrope HCl-eau. La concentration en acide demeure sensiblement constante par suite du dégagement d'acide chlorhydrique. On obtient un produit brut, avec un rendement de 88,5 %, celui en dichloro-2,6-nitro-4-aniline étant de 83,8 %. Les cristaux de produit ont une taille moyenne de 150 à 200 microns. On peut remarquer dans ce cas un allongement de la durée de chloration attribuable au dégagement d'acide chlorhydrique.

Exemple 5

On opère comme dans l'exemple 1 et on effectue la chloration à la température de 110 °C en maintenant dans l'appareil une légère pression de l'ordre de 0,3 bars. Cette surpression peut être facilement établie et maintenue en faisant le dégazage de l'acide chlorhydrique formé à travers une colonne barométrique d'acide chlorhydrique de hauteur convenable (environ 2,5 m).

La durée de chloration est ainsi réduite à moins de 3 heures. Le produit est isolé comme dans l'exemple 1 avec les mêmes résultats.

Exemple 6

Dans cet exemple et les exemples suivants, on effectue la chloration dans un autoclave fermé permettant de travailler sous pression.

Avec les mêmes charges que pour l'exemple 1, la durée d'introduction du chlore à 105 °C est ramenée à 1 heure 30 minutes. La pression dans l'appareil augmente jusqu'à 3 bars en fin de chloration. Après refroidissement, le produit est isolé comme dans l'exemple 1.

Exemple 7

La charge d'acide chlorhydrique étant faite à partir d'acide 7,5 N, la chloration est effectuée en introduisant le chlore à 110 °C. Lorsque la pression atteint 3 bars, on dégaze une partie de l'acide chlorhydrique jusque vers 2 bars et on reprend l'envoi de chlore. La chloration est terminée en moins de 2 h 30 minutes. L'appareil est refroidi, ramené à la pression atmosphérique en purgeant le gaz formé. Après filtration et lavage, on recueille 182 g de produit contenant :

90 % de dichloro-2,6-nitro-4-aniline
2,5 % de dichloro-2,4-nitro-6-aniline
1 % de chloro-2-nitro-4-aniline (rendement : 88 %).

**Revendications**

1. Procédé de fabrication de la dichloro-2,6-nitro-4-aniline par chloration avec du chlore gazeux, de la paranitroaniline, en solution aqueuse d'acide chlorhydrique, à l'ébullition, caractérisé en ce que la réaction est effectuée à une température de 95 à 110 °C, la concentration du milieu en acide chlorhydrique étant comprise entre 4 et 7,5 N.

2. Procédé selon la revendication 1, caractérisé en ce que la réaction est effectuée à 105-110 °C, la concentration du milieu en acide chlorhydrique étant comprise entre 4,5 et 6 N.

3. Procédé selon la revendication 1, caractérisé en ce que l'on opère avec une surpression.

4. Produit obtenu selon le procédé des revendications 1 à 3 se présentant sous forme de cristaux de taille moyenne comprise entre 100 et 300 microns.

**Claims**

1. A process for the manufacture of 2.6-dichloro-4-nitroaniline which comprises the chlorination, using chlorine gas, of para-nitroaniline in aqueous hydrochloric acid at the boil, characterized in that the reaction is carried out at a temperature of 95 °C to 110 °C, the concentration of hydrochloric acid in the reaction medium being from 4 to 7.5 N.

2. A process according to claim 1, characterized in that the reaction is carried out at 105-110 °C, the concentration of hydrochloric acid in the reaction medium being from 4.5 to 6 N.

3. A process according to claim 1, characterized in that the reaction is carried out under increased pressure.

4. Product obtained by the process according to claims 1 to 3 which is in the form of crystals having an average size of from 100 to 300 microns.

**Ansprüche**

1. Verfahren zur Herstellung von 2,6-Dichlor-4-nitroanilin durch Chlorierung von p-Nitroanilin in wäßriger Salzsäurelösung am Siedepunkt mit gasförmigem Chlor, dadurch gekennzeichnet, daß die Reaktion bei 95 bis 110 °C in einem Medium mit einer Salzsäurekonzentration von 4 bis 7,5 N vorgenommen wird.

2. Verfahren nach Anspruch 1, dadurch gekennzeichnet, daß die Reaktion bei 105 bis 110 °C in einem Medium mit einer Salzsäurekonzentration von 4,5 bis 6 N durchgeführt wird.

3. Verfahren nach Anspruch 1 oder 2, dadurch gekennzeichnet, daß die Reaktion unter Überdruck durchgeführt wird.

4. 2,6-Dichlor-4-nitroanilin, erhältlich nach dem Verfahren nach einem der Ansprüche 1 bis 3 in Form von Kristallen einer mittleren Größe von 100 bis 300 µm.